Europäisches Patentamt

European Patent Office

Office européen des brevets

(11) Veröffentlichungsnummer: **0 236 266 B1**

(12) **EUROPÄISCHE PATENTSCHRIFT**

(45) Veröffentlichungstag der Patentschrift: **19.06.91**

(21) Anmeldenummer: **87810077.5**

(22) Anmeldetag: **09.02.87**

(51) Int. Cl.⁵: **A61M 35/00**

(54) **Dermale und transdermale therapeutische Systeme mit musterförmig aufgetragener Klebschicht, sowie Verfahren zur dessen Herstellung.**

(30) Priorität: **14.02.86 US 829636**

(43) Veröffentlichungstag der Anmeldung:
**09.09.87 Patentblatt 87/37**

(45) Bekanntmachung des Hinweises auf die
Patenterteilung:
**19.06.91 Patentblatt 91/25**

(84) Benannte Vertragsstaaten:
**AT BE CH DE ES FR GB GR IT LI LU NL SE**

(56) Entgegenhaltungen:
**EP-A- 0 170 010**
**US-A- 3 598 122**
**US-A- 3 731 683**

(73) Patentinhaber: **CIBA-GEIGY AG**
**Klybeckstrasse 141**
**CH-4002 Basel(CH)**

(72) Erfinder: **Loveland, Frederic D.**
**13 Navajo Trail**
**North Branch New Jersey 08876(US)**

## Beschreibung

Die Erfindung betrifft ein dermales oder transdermales therapeutisches System in Form von Pflastern für die Applikation von Wirkstoffen, soure ein verfahren zur dessen Herstellung.

In der Europäischen Patentanmeldung 170 010 sind dermale therapeutische Systeme zur topischen Applikation von Wirkstoffen beschrieben, die eine punktförmige diskontinuierliche Klebschicht aufweisen.

Andere Pflaster kann man bezüglich Funktionsweise in zwei Kategorien einteilen. Die erste enthält den Wirkstoff oder die Wirkstofformulierung in der Klebschicht oder enthält die Wirkstofformulierung in einem Reservoir, welches mit der Klebschicht verschlossen ist. Zur zweiten Kategorie gehören solche Pflaster, welche den Wirkstoff oder die Wirkstofformulierung in einem Reservoir enthalten, in dessen Füllmaterial der Wirkstoff entweder gelöst ist oder sich in Hohlräumen wie Flüssigkeit in einem durchlässigen Schwamm befindet. In diesen Pflastern befindet sich die Klebschicht nur in ringförmiger Anordnung am Rande der Kontaktfläche zwischen Wirkstoffreservoir und der Haut des Patienten. Solche Pflaster sind in den U.S Patentschriften 3,598,122, 3,598,123, 3,731,683 3,734,097, 3,742,951 und 3,797,494 beschrieben.

Die bisher bekannten Pflaster haben gewisse Nachteile. Bei bestimmten Pflastern eignen sich nur wenige Klebermaterialien, da der Wirkstoff diese Klebschicht durchdringen muss, wenn er bis zur Haut gelangen soll.

Ist der Wirkstoff in der Klebschicht löslich, kann sich der Wirkstoff darin mit der Zeit anreichern. Beim Anbringen des Pflasters auf der Haut kann der Wirkstoff in einer erhöhten Initialdosis und später in geringer Menge an die Haut abgegeben werden. Das Abgabeverhalten dieser Darreichungsform ähnelt dann den üblichen oralen Darreichungsformen mit hoher Initialdosis und anschliessendem Konzentrationsabfall. Anderrerseits muss die Klebschicht für den Wirkstoff stets durchlässig sein, so dass unlösliche Klebermaterialien generell ausscheiden.

Die zur genannten zweiten Kategorie gehörenden Pflaster, worin die Klebschicht sich nur am Rande der Kontaktfläche zwischen Wirkstoffreservoir und der Haut des Patienten befindet, sind ebenfalls problematisch: Während der Applikationszeit besteht nicht immer der volle Kontakt zwischen der Kontaktfläche des Wirkstoffreservoirs und der Haut des Patienten. Dies hat zur Folge, dass der Sitz des Pflasters nicht gewährleistet ist und sich Falten und Knicke bilden. Ausserdem müssen im Verhältnis zur applizierbaren Wirkstoffmenge unverhältnismässig grosse Pflaster angebracht werden. Steht nur insgesamt eine kleine Gesamtfläche zur Verfügung, z.B. aus kosmetischen Gründen, wird ein zu hoher Anteil der Kontaktfläche für den Kleber benötigt. Will man mit weniger Klebstofffläche bzw. Klebstoffmenge auskommen, muss man Klebstoffe mit stärkerer Klebwirkung verwenden, was bei der Entfernung der Pflaster problematisch ist und Patienten zu nicht regelmässigem Gebrauch dieser Darreichungsform verleitet.

Der vorliegenden Erfindung liegt die Aufgabe zugrunde, transdermale oder dermale therapeutische Systeme in Form von Pflastern mit verbesserten Abgabeeigenschaften ohne die genannten Nachteile bereitzustellen.

Der vorliegenden Erfindung liegt ausserdem die Aufgabe zugrunde, transdermale therapeutische Systeme in Form von Pflastern mit bisher nicht dafür verwendeten Klebematerialien bereitzustellen.

Ebenfalls liegt der vorliegenden Erfindung die Aufgabe zugrunde, transdermale therapeutische Systeme in Form von Pflastern mit guter Haftwirkung auf der Haut und möglichst grosser Ausnutzung der Kontaktfläche bereitzustellen.

Diese Aufgaben werden in überraschender Weise gelöst, indem man ein streifenförmiges Klebstoffmuster auf der äusseren, dem Patienten zugewandten Seite des Wirkstoffreservoirs anbringt. Das Klebstoffmuster haftet auf der Haut des Patienten beim Anbringen des Pflasters.

Gegenstand der Erfindung ist ein dermales oder transdermales therapeutisches System in Form eines Pflasters bestehend aus einer in übereinander liegenden Schichten angeordneten und für die Komponenten der Wirkstofformulierung undurchlässigen Deckschicht , einem die Wirkstofformulierung beinhaltenden Reservoir, einer diskontinuierlichen Klebschicht auf der äusseren, dem Patienten zugewandten Seite des Reservoirs sowie einer abziehbaren Schutzschicht auf der Klebschicht. Das System ist dadurch gekennzeichnet, dass die Klebschicht aus einem streifenförmigen Klebstoffmuster und gegebenenfalls einem diskontinuierlichen oder kontinuierlichen Klebstoffrand als Begrenzung des Klebstoffmusters besteht.

Gegenstand der Erfindung ist ferner ein Verfahren zur Herstellung eines dermalen oder trasdermalen therapeutischen Systems in Form eines Pflasters bestehend aus einer in übereinander liegenden Schichten angeordneten und für die Komponenten der Wirkstofformulierung undurchlässigen Deckschicht, einem die Wirkstofformulierung beinhaltenden Reservoir, einer diskontinuierlichen Klebschicht auf der äusseren, dem Pateinten zugewandten Seite des Reservoirs sowie einer abziehbaren Schutzschicht auf der Klebschicht, dadurch gekennzeichnet, dass man die diskontinuierliche Klebschicht als streifenförmiges Muster und gegebenenfalls einen diskontinuierlichen oder Kontinuierlichen Klebstoffrand als Begrenzung des Musters auf der abziehbaren Schutzschicht aufträgt, darauf die Bestandteile des Reservoirs aufträgt, darauf die undurchlässige Deckschicht legt, die Schichten, und und das Reservoir verklebt oder verschweisst und das Pflaster ausstanzt.

Vorteilhafte Weiterbildungen der Erfindung sind in den abhängigen Ansprüchen definiert.

## Beschreibung der Zeichnungen

Fig. 1 zeigt ein erfindungsgemässes Pflaster ohne Schutzschicht mit Blick von oben.

Fig. 2 (Membransystem mit Schutzschicht) und Fig. 6 (Matrixsystem mit Schutzschicht) zeigen die Ausführungsform gem. Fig 1 im Querschnitt.

Fig. 3 zeigt eine weitere Ausführungsform des erfindungsgemässen Pflasters ohne Schutzschicht mit Blick von oben.

Fig. 4 zeigt eine weitere mögliche Anordnung der Klebschicht auf dem Wirkstoffreservoir.

Fig. 5 (Membransystem mit Schutzschicht) und Fig. 7 (Matrixsystem mit Schutzschicht) zeigen die Ausführungsform gem. Fig. 3 im Querschnitt.

## Beschreibung bekannter Ausführungsformen und Erklärung der Bezugszeichen 1-6

Bekannte dermale oder transdermale therapeutische Systeme in Form von Pflastern (1) sind in übereinander liegenden Schichten gleicher oder unterschiedlicher Grösse angeordnet und bestehen aus einer geschlossenen und für die Komponenten der Wirkstofformulierung undurchlässigen Deckschicht (2), einem die Wirkstofformulierung beinhaltenden Reservoir (3), einer diskontinuierlichen Klebschicht (4) und einer abziehbaren Schutzschicht (6) auf der Klebschicht.

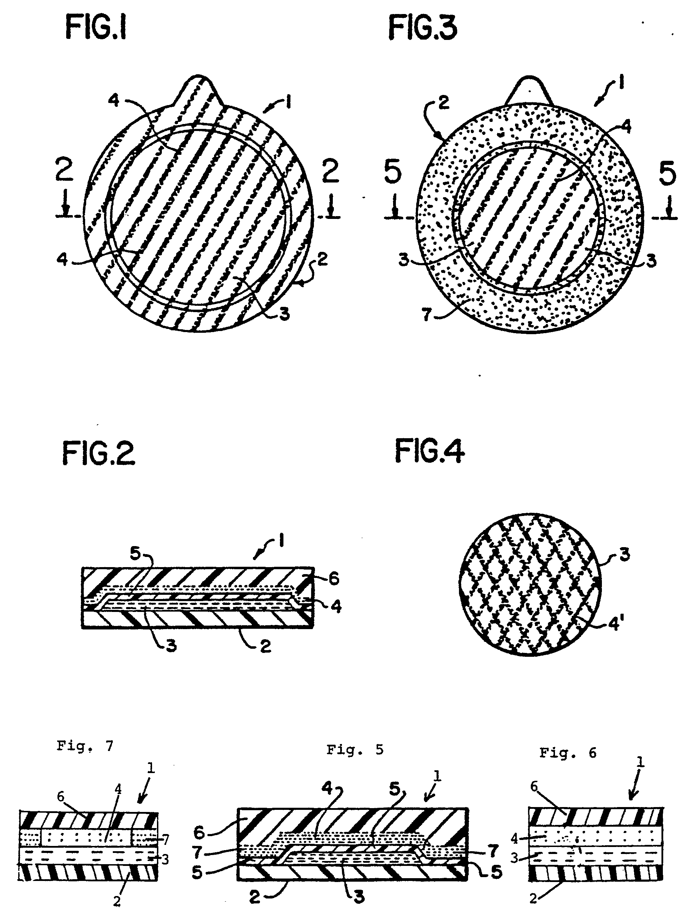

Das Reservoir (3) enthält Wirkstofformulierung, welche entweder in einem polymeren Matrixmaterial eingebettet ist (Matrixsystem) oder durch eine Membranschicht (5) abgeschlossen wird, durch welche der Wirkstoff wandern kann. Die Deckschicht (2) besteht aus Material, welches für die Komponenten der Wirkstofformulierung undurchlässig ist und bedeckt die äussere Seite des Reservoirs (3), welche der Applikationsfläche (Haut des Patienten) abgewandt ist. Wenn die Deckschicht (2) dieselben Flächenmasse wie das Reservoir (3) einnimmt, wird dessen äussere Seite nur von oben bedeckt, wobei das Reservoir am Rand offen bleibt, siehe Figuren 6 und 7.

Nimmt die Deckschicht (2) grössere Flächenmasse als das Reservoir (3) ein, ist der überstehende Teil der Deckschicht (2) entweder mit der Klebschicht (4) oder mit der Membran (5) verbunden, siehe Figuren 2 und 5. So können die Deckschicht (2) und die Membran (5) eine dazwischen liegende abgeschlossene Kammer für die Wirkstofformulierung bilden (Wirkstoffreservoir). Pflaster, worin nur die Ränder des Reservoirs oder die ganze dem Anwendungsort zugewandte Fläche mit Klebstoff bedeckt sind, sind in der U.S. Patentschrift 3,797,494 beschrieben.

## Beschreibung der erfindungsgemässen Ausführungsform und Erklärung des Bezugszeichens 7

Bei der erfindungsgemässen Ausführungsform ist die dem Anwendungsort, z.B. der Haut des Patienten, zugewandte äussere Fläche des Reservoirs (3) mit einer diskontinuierlichen Klebschicht (4) in streifenförmiger Anordnung des Klebstoffmusters mit Zwischenräumen bedeckt. Die streifenförmige Anordnung des Klebstoffs kann durch einen oder mehrere diskontinuierliche oder vorzugsweise kontinuierliche Klebstoffränder (7) begrenzt werden. In einer bevorzugten Ausführungsform der Erfindung ist die Membran (5) durch eine streifenförmige Klebschicht in musterförmiger Anordnung mit gleichen Zwischenräumen bedeckt. Die musterförmige Anordnung wird durch einen kontinuierlichen Klebstoffrand (7) begrenzt, welcher den äusseren Rand der Membran (5) bedeckt.

Die geometrische Form des erfindungsgemässen Pflasters, die Form und Anordnung des Reservoirs (3) sowie dessen Inhalt können beliebig gestaltet werden und sind nicht auf die in den Abbildung gezeigten Raumformen beschränkt.

Beispielsweise kann das in Fig. 4 gezeigte Klebstoffmuster 4' gewählt werden. Bevorzugt sind Muster mit regelmässiger Anordnung mit Zwischenräumen in gleichmässigen Abständen. Ebenfalls ist die Methode zum Auftragen des Klebstoffmusters beliebig. Bevorzugt sind Druckmuster.

Mindestens 20 %, vorzugsweise mindestens 30 %, insbesondere mindestens 40 %, der gesamten Kontaktfläche und vorzugsweise der Berührungsfläche des Reservoirs (3) sollten mit Klebstoff bedeckt sein, um einen einwandfreien Sitz zu gewährleisten. So sind nicht mehr als 80 % vorzugsweise nicht mehr als 60 %, insbesondere nicht mehr als 40 %, der gesamten äusseren und der Applikationsfläche zugewandten Fläche des Reservoirs (3) mit Klebstoff bedeckt.

Die Klebschicht (4) kann ausserdem den Rand (7) bilden oder das streifenförmige Muster auf dem Wirkstoffreservoir umrunden und als diskontinuierliches oder vorzugsweise kontinuierliches Band auf der äusseren dem Anwendungsort zugewandten Seite des Reservoirs (3) angebracht sein, siehe Fig. 3. Bei solchen Ausführungsformen besteht die gesamte Klebschicht (4) aus einer Kombination des streifenförmigen Musters auf dem Reservoir (3) mit einem dieses Muster umgebenden kontinuierlichen Band. Die Form dieses Bandes am Rand ist beliebig und von der Raumform des Pflasters gegeben und ist vorzugsweise rund oder rechteckig. Das Band am Rand aus Klebstoff ist ein bevorzugtes Merkmal, da dieses das Pflaster mit der Haut siegelförmig verschliesst und so das Austreten von Bestandteilen der Wirkstofformulierung verhindert. Ist ein kontinuierliches Band aus Klebematerial am Rande der Aussenseite des Reservoirs (3) vorhanden, brauchen nur ca. 10 % der Oberfläche des Reservoirs (3), welche die Berührungsfläche bildet, mit Klebstoff bedeckt sein. Die Breite des umgebenden diskontinuierlichen oder kontinuierlichen Bandes ist beliebig.

Die für die erfindungsgemässen dermalen oder transdermalen therapeutischen Systeme (1) verwendbaren Klebermaterialien müssen sich für die Verwendung auf der Haut eignen. Ausserdem dürfen sie nicht mit den Bestandteilen des Reservoirs (3) oder der Deckschicht (2) sowie mit dem Wirkstoff selbst reagieren. Klebematerialien, welche solche Anforderungen erfüllen, sind bekannt.

Die Klebematerialien lassen sich aufgrund der Löslichkeit der Wirkstoffe darin in drei Gruppen einteilen: (1) verschliessend oder nicht aufnahmefähig, (2) aufnahmefähig oder löslich und (3) kaum aufnahmefähig oder löslich. Für den Wirkstoff Nitroglycerin sind nicht aufnahmefähige Klebermaterialien beispielsweise: Natürlicher oder synthetischer Kautschuk auf der Basis Styrolbutadien, Polyisobutylen, Polybutadien, Polyisopren und Blockcopolymere damit. Für Nitroglycerin aufnahmefähige Klebermaterialien sind beispielsweise: Acryl- oder Methacrylsäureharze, Polyurethane, Vinylpolymere, Aethylen-Vinylacetatcopolymere mit Kleberharzzusätzen. Auch gering aufnahmefähige Klebermaterialien können verwendet werden. Die obige Einteilung ist willkürlich und von der Löslichkeit des Wirkstoffs im Klebermaterial abhängig. Die Wahl des betreffenden Klebermaterials wird von der Aufgabenstellung abhängen, ob eine mehr oder minder grosse Initialdosis des darin gelösten Wirkstoffs gewünscht oder nachteilig ist.

Bevorzugt sind Klehermaterialien auf der Basis Polystyrol, z.B. Kraton® (Shell) Copolymere, Aethylen-Vinylacetat-Copolymere, z.B. Elvax® (Du Pont)- oder Vynathene (U.S.I. Chemicals)-Typen, Polybutadien, z.B. Indopol® (Amoco), Polyisobutylen, z.B. Oppanol® (BASF) oder Acrylsäure-oder Methacrylsäure, z.B. Acronal® (BASF), Homopolymere, Siliconkautschuk, z.B. DC Medical Adhesive (Dow), Vinylpolymere oder Polyurethane, z.B. Vondic® (Dainippon). Die genannten Kleber sind Rezepturen mit Bezeichnung des Herstellers basierend auf den genannten Basispolymeren.

Die erfindungsgemässen dermalen und transdermalen therapeutischen Systeme werden hergestellt, indem man die diskontinuierliche Klebschicht (4) als streifenförmiges Muster und gegebenenfalls einen diskontinuierlichen oder kontinuierlichen Klebstoffrand (7) als Begrenzung des Musters auf der abziehbaren Schutzschicht (6) aufträgt, darauf die Bestandteile des Reservoirs (3) aufträgt, darauf die undurchlässige Deckschicht (2) legt, die Schichten (2), (4) und (6) und das Reservoir (3) verklebt oder verschweisst und das Pflaster ausstanzt. Gegebenenfalls kann das Reservoir (3) mit zusätzlichem Klebstoff mit der Deckschicht (2) verbunden werden. Ebenso kann das Reservoir (3) mit der Membranschicht (5) oder mit der Klebschicht (4) heiss verschweisst werden. Bei Flüssigkeits-gefüllten Systemen wird die Membranschicht (5) auf die Klebschicht (4) aufgetragen und auf die Membran (5) die Wirkstofformulierung gebracht. Die Herstellungsverfahren sind den bekannten Methoden, welche z.B. in der U.S. Patentschrift 3,797,494 beschrieben sind, analog.

Für die erfindungsgemässen therapeutischen Systeme können beliebige Wirkstoffe verwendet werden. Insbesondere können systemische Wirkstoffe in der vorgesehenen Dosierung für die bekannten therapeutischen Verfahren verwendet werden. Geeignete systemische Wirkstoffe sind unter anderem Antibiotika aus der Gruppe der Penicilline, Tetracyclin, Oxytetracylin, Chlortetracylin, Chloramphenicol, Cephalosporine, Peneme, Aminoglycoside und Sulfonamide, Sedativa wie Pentabarbital-Natrium, Codein, Bromisovalerylharnstoff, Carbromal und Natriumphenobarbital, Stimulanzien mit Wirkung auf das zentrale Nervensystem wie 3-(2-Amino-propyl)indolacetat oder 3-(2-Aminobutyl)indolacetat, Beruhigungsmittel wie Reserpin, Chlorpromazinhydrochlorid oder Thiopropazathydrochlorid, Hormone vom Adrenocorticosteroid-Typ wie Methylprednisolon, androgene Steroide wie Methyltestosteron oder Fluoxymesteron, oestrogene Steroide wie Oestron, 17$\beta$-Oestradiol oder Aethnylöstradiol, progestagene Steroide wie 17$\alpha$-Acetoxyprogesteron, Medroxyprogesteronacetat, 19-Norprogesteron oder Norethindron, Schilddrüsenhormone wie Thyroxin, Antipyretika wie Aspirin, Salicylamid oder Natriumsalicylat, Morphin und andere Analgetika auf Morphinbasis, Antidiabetika wie Insulin, Herz-Kreislaufmittel wie Nitroglycerin, Betablocker wie Metoprolol oder Oxprenolol, Digitalispräparate wie Digitoxin, Digoxin oder Ouabain, Anticholinergika wie Atropin, Scopolamin-hydrobromid, Methscopolaminbromid oder Hyoscyamin, Antimalariamittel aus der Gruppe der 4-Aminochinoline, 9-

4

Aminochinoline sowie Pyrimethamin, Nahrungsmittelzusätze wie Vitamine, essentielle Aminosäuren oder essentielle Fette.

Statt der genannten systemischen Wirkstoffe können auch topische Wirkstoffe in der vorgesehenen Dosierung für die bekannten therapeutischen Verfahren verwendet werden. Geeignete topische Wirkstoffe sind beispielsweise Antiperspiranzien wie Aluminiumchlorid, Deodoranzien wie Triclosan oder Methylbenzethoniumchlorid, adstringierende Mittel wie Tanninsäure, Irritanzien wie Methylsalicylat oder Kampher, Kantharidine, Keratolytika wie Benzoesäure, Salicylsäure, Resorcin, Iodochlorhydroxyquin, antifungische Mittel wie Tolnaftat, Griseofulvin, Mystatin oder Amphotericin, Antiinflammatorika vom Corticosteroidtyp, z.B. Hydrocortison, Hydrocortisonacetat, Prednisolon, Methylprednisolon, Triamcinolon-acetonid, Fludrocortison, Flurandrenolon, Flumethason, Dexamethason, Betamethason, Fluocinolonacetonid, Fluorometholon oder Pramoxin, Antineoplastika, z.B. Methotrexat, antibakterielle Mittel wie Bacitracin, Neomycin, Erythromycin, Tetracyclin, Chlortetracyclin, Chloramphenicol, Oxytetracyclin, Polymycin B, Nitrofuraxon, Mafenid, Benzalkoniumchlorid, Cetalkoniumchlorid, Methylbenzethoniumchlorid oder Neomycinsulfat.

Von den genannten Wirkstoffen sind Nitroglycerin, Scopolamin, $17\beta$-Oestradiol und Arecholin besonders bevorzugt.

Die Einteilung der genannten Wirkstoffe in systemische oder topische Wirkstoffe ist willkürlich. Sehr viele Wirkstoffe können sowohl systemisch als auch topisch wirksam sein. Für die topische Wirkung können beispielsweise geringere Mengen als für die systemische Wirkung benötigt werden, ausreichen. Durch Einsatz eines Penetrationsverbesserers wie Azon® können andererseits Wirkstoffe mit topischer Dosierung für einen systemischen Effekt verwendet werden.

Statt der genannten Wirkstoffe selbst bzw. statt der genannten Salze können die betreffenden Salze bzw. freien Säuren oder Basen sowie einfache Derivate davon wie Ester, Amide, Acetale oder Aether verwendet werden. Statt der Wirkstoffe selbst kann man bevorzugt Wirkstofformulierungen, welche die gewünschten Permeationseigenschaften oder die gewünschte Transportgeschwindigkeit gewährleisten, verwenden. Statt der Wirkstoffe selbst kann man auch Kombinationen davon verwenden. Bei Einsatz von Derivaten muss die Umwandlung in den freien Wirkstoff, z.B. durch Einwirkung von Enzymen, gewährleistet sein.

Das folgende Bespiel soll die Erfindung illustrieren ohne deren Umfang zu beschränken.

Beispiel

Man stellt transdermale therapeutische Systeme in Form von Pflastern mit Nitroglycerin als Wirkstoff her:

Die Klebschicht wird in einer musterförmigen Anordnung mit gleichmässigen Zwischenräumen aufgetragen. Die Bedeckung mit Klebstoff ist daher geringer als 100 %. Die hergestellten Pflaster unterscheiden sich durch die verwendeten Klebermaterialien und underschiedlichen Bedeckungsgrad der Kontaktfläche auf der Haut des Patienten. 100 %ige Bedeckung entspricht den Systemen des Standes der Technik (Kontrollsystem in der Tabelle). Als Kontrollsystem wird in der Tabelle Transderm® Nitro-5, ein Handelsprodukt von Ciba-Geigy mit 100 %iger Bedeckung angeführt. Die kumulierten Abgabemengen an Wirkstoff für jedes Systems sind in der beliegenden Tabelle angeführt.

ABGABEMENGEN VOM NITROGLYCERIN ALS FUNKTION DER ZEIT IN VERSCHIEDENEN TTS-SYSTEMEN

| TTS-System | Kumulative Nitroglycerin Abgabemenge ($\mu g/cm^2$) | | | | | | |
|---|---|---|---|---|---|---|---|
| | 1 h | 4 h | 8 h | 12 h | 16 h | 20 h | 24 h |
| Transderm® Nitro 5 (Mittelwert gebildet aus 3 verschiedenen Produktproben) | 111 | 303 | 532 | 748 | 960 | 1160 | 1348 |
| Medical Adhesive Modified S-15, | | | | | | | |
| 100 % Bedeckung | 101 | 290 | 521 | 747 | 961 | 1156 | 1354 |
| 40 % Bedeckung | 103 | 266 | 469 | 673 | 862 | 1052 | 1230 |
| 20 % Bedeckung | 81 | 260 | 486 | 693 | 886 | 1059 | 1234 |
| Century CA-1028 (Acrylic), | | | | | | | |
| 100 % Bedeckung | 398 | 609 | 800 | 971 | 1082 | 1275 | 1358 |
| 40 % Bedeckung | 373 | 746 | 1004 | 1200 | 1378 | 1546 | 1701 |
| 20 % Bedeckung | 181 | 439 | 690 | 906 | 1109 | 1289 | 1454 |
| Fuller HM-6677 (Kraton) | | | | | | | |
| 100 % Bedeckung | 35 | 63 | 83 | 110 | 131 | 149 | 167 |
| 40 % Bedeckung | 85 | 241 | 436 | 620 | 793 | 963 | 1118 |
| 20 % Bedeckung | 66 | 224 | 420 | 604 | 783 | 948 | 1106 |
| Century GI-1 (Kraton) | | | | | | | |
| 100 % Bedeckung | 3 | 9 | 17 | 23 | 29 | 34 | 34 |
| 40 % Bedeckung | 65 | 196 | 350 | 498 | 637 | 772 | 902 |
| 20 % Bedeckung | 90 | 271 | 500 | 712 | 918 | 1113 | 1301 |

## Ansprüche

1. Dermales oder transdermales therapeutisches System (1) in Form eines Pflasters bestehend aus einer in übereinander liegenden Schichten angeordneten und für die Komponenten der Wirkstofformulierung undurchlässigen Deckschicht (2), einem die Wirkstofformulierung beinhaltenden Reservoir (3), einer

EP 0 236 266 B1

diskontinuierlichen Klebschicht (4) auf der äusseren, dem Patienten zugewandten Seite des Reservoirs sowie einer abziehbaren Schutzschicht (6) auf der Klebschicht, dadurch gekennzeichnet, dass die Klebschicht (4) aus einem streifenförmigen Klebstoffmuster und gegebenenfalls einem diskontinuierlichen oder kontinuierlichen Klebstoffrand (7) als Begrenzung des Klebstoffmusters besteht.

2. Dermales oder transdermales therapeutisches System gemäss Anspruch 1, dadurch gekennzeichnet, dass das Klebstoffmuster durch einen kontinuierlichen Klebstoffrand (7) begrenzt wird.

3. Dermales oder transdermales therapeutisches System gemäss Anspruch 1 oder 2, dadurch gekennzeichnet, dass das Reservoir durch eine Membran (5) bedeckt wird und diese Membran ihrerseits durch das Klebstoffmuster bedeckt wird, wobei das Klebstoffmuster aus Zwischenräumen mit gleichen Abständen und kontinuierlichem Klebstoffrand (7) bedeckt wird.

4. Dermales oder transdermales therapeutisches System gemäss Anspruch 3, dadurch gekennzeichnet, dass der Klebstoffrand (7) ringförmig ist.

5. Dermales oder transdermales therapeutisches System gemäss Anspruch 1 oder 2, dadurch gekennzeichnet, dass das Klebstoffmuster aus einer streifenförmigen Anordnung mit Zwischenräumen mit gleichen Abständen besteht.

6. Verfahren zur Herstellung eines dermalen oder transdermalen therapeutischen Systems (1) in Form eines Pflasters bestehend aus einer in übereinander liegenden Schichten angeordneten und für die Komponenten der Wirkstofformulierung undurchlässigen Deckschicht (2), einem die Wirkstofformulierung beinhaltenden Reservoir (3), einer diskontinuierlichen Klebschicht (4) auf der äusseren, dem Patienten zugewandten Seite des Reservoirs sowie einer abziehbaren Schutzschicht (6) auf der Klebschicht, dadurch gekennzeichnet, dass man die diskontinuierliche Klebschicht (4) als streifenförmiges Muster und gegebenenfalls einen diskontinuierlichen oder kontinuierlichen Klebstoffrand (7) als Begrenzung des Musters auf der abziehbaren Schutzschicht (6) aufträgt, darauf die Bestandteile des Reservoirs (3) aufträgt, darauf die undurchlässige Deckschicht (2) legt, die Schichten (2), (4) und (6) und das Reservoir (3) verklebt oder verschweisst und das Pflaster ausstanzt.

7. Verfahren zur Herstellung eines dermalen oder transdermalen therapeutischen Systems gemäss Anspruch 6, dadurch gekennzeichnet, dass man einen kontinuierlichen Klebstoffrand (7) als Begrenzung des Musters aufträgt.

8. Verfahren zur Herstellung eines dermalen oder transdermalen therapeutischen Systems gemäss Anspruch 6 oder 7, dadurch gekennzeichnet, dass man ein streifenförmiges Klebstoffmuster mit gleichen Abständen und einem kontinuierlichen Klebstoffrand (7) aufträgt.

9. Verfahren zur Herstellung eines dermalen oder transdermalen therapeutischen Systems gemäss Anspruch 8, dadurch gekennzeichnet, dass man einen engförmigen Klebstoffrand (7) aufträgt.

**Claims**

1. A dermal or transdermal therapeutic system (1) in the form of a patch consisting of a backing layer (2) that is impermeable to the components of the active ingredient formulation and is arranged in layers lying one on top of another, and a reservoir (3) containing the active ingredient formulation, a discontinuous adhesive layer (4) on the external side of the reservoir towards the patient, and a removable protective layer (6) on the adhesive layer, characterised in that the adhesive layer (4) consists of a strip-shaped pattern of adhesive and, optionally, a discontinuous or continuous border of adhesive (7) as a boundary for the pattern of adhesive.

2. A dermal or transdermal therapeutic system according to claim 1, characterised in that the pattern of adhesive is bounded by a continuous border of adhesive (7).

3. A dermal or transdermal therapeutic system according to either claim 1 or claim 2, characterised in that the reservoir is covered by a membrane (5) and that membrane is in turn covered by the pattern of adhesive, wherein the pattern of adhesive consisting of spaces arranged at uniform intervals and a

7

EP 0 236 266 B1

continuous border of adhesive (7) is covered.

4. A dermal or transdermal therapeutic system according to claim 3, characterised in that the border of adhesive (7) is annular.

5. A dermal or transdermal therapeutic system according to either claim 1 or claim 2, characterised in that the pattern of adhesive consists of a strip-shaped arrangement with spaces arranged at uniform intervals.

6. A process for the manufacture of a dermal or transdermal therapeutic system (1) in the form of a patch consisting of a backing layer (2) that is impermeable to the components of the active ingredient formulation and is arranged in layers lying one on top of another, and a reservoir (3) containing the active ingredient formulation, a discontinuous adhesive layer (4) on the external side of the reservoir towards the patient, and a removable protective layer (6) on the adhesive layer, characterised in that the discontinuous adhesive layer (4) is applied to the removable protective layer (6) in the form of a strip-shaped pattern and, optionally, a discontinuous or continuous border of adhesive (7) as a boundary for the pattern, the components of the reservoir (3) are applied thereto, the impermeable backing layer (2) is laid thereon, the layers (2), (4) and (6) and the reservoir (3) are adhesively bonded or welded together and the patch is punched out.

7. A process for the manufacture of a dermal or transdermal therapeutic system according to claim 6, characterised in that a continuous border of adhesive (7) is applied as the boundary for the pattern.

8. A process for the manufacture of a dermal or transdermal therapeutic system according to either claim 6 or claim 7, characterised in that a strip-shaped pattern of adhesive at uniform intervals and a continuous border of adhesive (7) are applied.

9. A process for the manufacture of a dermal or transdermal therapeutic system according to claim 8, characterised in that a narrow border of adhesive (7) is applied.

**Revendications**

1. Système thérapeutique dermique ou transdermique (1) sous forme d'un emplâtre, constitué d'une couche de recouvrement (2) disposée en couches superposées et imperméable pour les composants de la formulation de la substance active, d'un réservoir (3) contenant la formulation de la substance active, d'une couche adhésive (4) discontinue sur la face extérieure, tournée vers le patient, du réservoir et d'une couche protectrice (6) pouvant être enlevée sur la couche adhésive, caractérisé en ce que la couche adhésive (4) est constituée d'un modèle formant des bandes réalisé par l'adhésif et éventuellement d'un bord adhésif (7) continu ou discontinu, délimitant le modèle réalisé par l'adhésif.

2. Système thérapeutique dermique ou transdermique selon la revendication 1, caractérisé en ce que le modèle réalisé par l'adhésif est délimité par un bord adhésif (7) continu.

3. Système thérapeutique dermique ou transdermique selon la revendication 1 ou 2, caractérisé en ce que le réservoir est recouvert d'une membrane (5) et en ce que cette membrane est recouverte, de son côté, par le modèle réalisé par l'adhésif, ledit modèle comportant des espaces présentant les mêmes écartements et étant recouvert d'un bord adhésif (7) continu.

4. Système thérapeutique dermique ou transdermique selon la revendication 3, caractérisé en ce que le bord adhésif (7) est annulaire.

5. Système thérapeutique dermique ou transdermique selon la revendication 1 ou 2, caractérisé en ce que le modèle réalisé par l'adhésif est constitué d'une disposition en forme de bandes avec des espaces ayant les mêmes écartements.

6. Procédé pour la préparation d'un système thérapeutique dermique ou transdermique (1) sous forme d'un emplâtre, constitué d'une couche de recouvrement (2) disposée en couches superposées et imperméable pour les composants de la formulation de la substance active, d'un réservoir (3) contenant

8

la formulation de la substance active, d'une couche adhésive (4) discontinue sur la face extérieure, tournée vers le patient, du réservoir et d'une couche protectrice (6) pouvant être enlevée sur la couche adhésive, caractérisé en ce que l'on applique, sur la couche protectrice (6) pouvant être enlevée, la couche adhésive (4) discontinue sous la forme d'un modèle formant des bandes et éventuellement un bord adhésif (7) continu ou discontinu, délimitant le modèle, en ce que l'on applique ensuite les constituants du réservoir (3), en ce que l'on place ensuite la couche de recouvrement (2) imperméable, en ce que l'on colle ou l'on soude les couches (2), (4) et (6) et le réservoir (3) et en ce que l'on découpe l'emplâtre à la matrice.

7. Procédé pour la préparation d'un système thérapeutique dermique ou transdermique selon la revendication 6, caractérisé en ce que l'on applique un bord adhésif (7) continu, délimitant le modèle.

8. Procédé pour la préparation d'un système thérapeutique dermique ou transdermique selon la revendication 6 ou 7, caractérisé en ce que l'on applique un modèle formant des bandes réalisé par l'adhésif présentant les mêmes écartements et un bord adhésif (7) continu.

9. Procédé pour la préparation d'un système thérapeutique dermique ou transdermique selon la revendication 8, caractérisé en ce que l'on applique un bord adhésif (7) étroit.

# FIG.1

# FIG.3

# FIG.2

# FIG.4

Fig. 7

Fig. 5

Fig. 6